# EUROPEAN PATENT APPLICATION

(11) **EP 3 269 357 A1**
(43) Date of publication of application: **17.01.2018**
(21) Application number: 16179750.1
(22) Date of filing: 15.07.2016
(51) Int. Cl.: A61K 9/00, A61K 47/02, A61K 9/51, A61K 33/08, A61K 33/24, A61K 33/26, A61K 33/32, C01G 45/02

(54) **PARTICLES COMPRISING METALS AND/OR METAL OXIDES FOR USE TO TRANSFORM COMPOUNDS IN VIVO**

(71) Applicant: Universiteit Gent, 9000 Gent (BE)
(72) Inventor: The designation of the inventor has not yet been filed

(57) **Abstract**

The present invention relates to the usage of particles which comprise metals and/or metal oxides to reduce the amount of specific compounds in vivo. More specifically, and as an example, the present invention discloses that microparticles comprising manganese oxide can reduce the amount of hydrogen sulfide (H₂S) in the gastrointestinal tract of patients suffering from the presence of too much hydrogen sulfide without releasing any (potentially toxic) manganese in their intestinal tract. In other words, the present invention discloses particles which can -for example- be used to treat excess flatulence or irritable bowel syndrome and/or inflammatory bowel diseases.

## Description

### Technical field of the invention

The present invention relates to the usage of particles which comprise metals and/or metal oxides to reduce the amount of specific compounds in vivo. More specifically, and as an example, the present invention discloses that microparticles comprising manganese oxide can reduce the amount of hydrogen sulfide (H₂S) in the gastrointestinal tract of patients suffering from the presence of too much hydrogen sulfide without releasing any (potentially toxic) manganese in their intestinal tract. In other words, the present invention discloses particles which can -for example- be used to treat excess flatulence or irritable bowel syndrome and/or inflammatory bowel diseases.

### Background art

Hydrogen sulfide, as an example of a molecule which can be oxidized in vivo by a coated microparticle, is produced in the human or animal gut by sulfur-reducing bacteria and increases sensitivity to inflammation in the intestines (Devkota et al. Nature, 2012), flatulence and discomfort due to bloating. Specific categories of patients affected include those with Crohn's disease, ulcerative colitis and irritable bowel syndrome (IBS) associated with small intestinal bowel overgrowth (SIBO) of colonic bacteria. There is today no specific solution that targets removal of hydrogen sulfide from the gut.

In the case of bloating and discomfort for IBS/SIBO, activated carbon or the antibiotic rifaximin is often the prescribed therapy. Activated carbon aims to absorb chemical byproducts of bacteria, however it is very non-specific (it sorbs many other compounds, e.g. other drugs the patient has to take, food nutrients...) and it is recommended not to take this compound continuously. Rifaximin aims to eliminate the problem at the source by killing the offending bacteria, however this is not specific and instead eliminates all bacteria good and bad, and the dosing is long-term and often consisting of repeated doses, which can lead to bacterial antibiotic resistance, as well as long-term changes in the normal intestinal microflora. For clinical inflammation such as in Crohn's disease and ulcerative colitis, immuno-therapy such as anti-TNFa antibodies or corticosteroids are commonly prescribed, which have significant side effects that often lead patients to stopping therapy.

WO2006102536 also describes the treatment of a wide array of diseases and physiological conditions based on modulating the level of bacteria-derived hydrogen sulfide (H₂S) in the body. The latter document provides therapeutic agents which are capable of eradicating the bacterial overgrowth in order to reduce the amount of bacteria derived H₂S in a patient in need thereof. However, the latter document is silent about using coated particles comprising metals to specifically reduce H₂S levels in vivo.

Mitsui et al. (Clinica Chimica Acta, 2003) disclose the effects of nitrate and metals such as bismuth, iron and zinc on free sulfide (H₂S) in batch cultures seeded with fecal flora. However, these authors conclude that the latter compounds are effective at reducing free H₂S concentrations but that toxic side effects of these metals and nitrate may seriously limit their use in vivo.

There is thus no specific and non-toxic solution for taking away only hydrogen sulfide, or any other compound, from the gut.

It is known that sulfide can be oxidized at carbon electrodes (Dutta, P. K., K. Rabaey, Z. G. Yuan, and J. Keller. 2008. Spontaneous electrochemical removal of aqueous sulfide. Water Research 42:4965-4975). This implies that a carbon material is poised at a sufficiently positive potential, e.g. 0 mV versus a Ag/AgCl reference electrode, causing oxidation of the sulfide to e.g. elemental sulfur. Without poising the potential, which implies connection to an electric circuit, carbon by itself will not transform sulfide.

Similarly, besides reduced compounds such as hydrogen sulfide which require oxidation, other compounds may require oxidation in order to decrease toxicity or to improve well-being of the host. For example, hydrogen peroxide can exert toxicity via three main mechanisms: corrosive damage, oxygen gas formation and lipid peroxidation (Watt BE, Proudfoot AT, Vale JA. Toxicol Rev. 2004;23(1):51-7).

Further, organic transformations occur in the gut, which can lead to both positive or negative health effects. Such organic transformations are typically mediated by enzymatic reactions, requiring besides the enzyme in many cases an electron donor/acceptor and a redox cofactor. In the field of bioelectrochemistry, such enzymes can be immobilized on carbon electrodes, and via a redox mediator and/or cofactor electricity is used as driver of specific enzymatic reactions. An example of this in practice is the measurement of ethanol which relies on its enzymatic conversion at an electrode (Martha Lee Weeks, Design of a Reagentless Enzymatic Amperometric Alcohol Biosensor: Yeast Alcohol Dehydrogenase and Nicotinamide Adenine Dinucleotide on Vertically Aligned Carbon Nanofibers, PhD thesis, University of Tennessee, 8/2006)

### Brief description of figures

Figure 1: Dissolution reaction mechanism of hydrogen sulphide oxidation onto manganese oxide. Firstly formation of complex manganese-sulfur occurs, subsequent manganese oxidation occurs after which either the sulfur pathway (left) or sulfate pathway (right) can occur.
Figure 2: Scanning electron microscope picture of carbon-coated MnO₂ microparticles.
Figure 3: HS⁻ (**a**) and H₂S (**b**) removal from liquid and gas phase, respectively, using carbon-coated MnO₂ particles in phosphate buffer pH 5.8.
Figure 4: (**a**) released of Mn in solution using carbon-coated MnO₂ versus MnO₂ particles. (**b**) growth curve of *Faecalibacterium prausnitzii* with versus without carbon-coated MnO₂ particles.

### Summary of the invention

The present invention relates to a safe, low cost solution that will take away sulfide -as an example-specifically from the gut under the form of elemental sulfur. There is no microbial resistance build-up as antibiotics are avoided and the product can be taken continuously by the patient either separately in pill form or blended into food. Moreover, the present invention can also be applied to many other processes occurring in the gut, both oxidative and reductive, and can be extended to the companion animal market. Furthermore, all of the aforementioned, current therapies have been shown to impact the gut microbiota in ways that often prevent restoration of the normal flora, or lead to overgrowth of harmful species. The particles of the present invention pass through the GI tract with limited impact on the gut microbiota and without releasing metal/metal oxides in the gut.

In one embodiment, the present invention thus relates to oxidative particles, such as manganese dioxide or iron oxide, which is coated with a conductive and porous layer which can consist of carbon. These particles are at micrometer or sub-micrometer (nanometer) scale.

The oxidative particle is ingested (1 gram contains about 1 trillion particles if the size is set at 1 micrometer), does not disturb the natural gut microbiota and does minimally release metal/metal oxides in the gut. In situ hydrogen sulfide, either from the aqueous matrix or diffused from the gaseous matrix into the aqueous phase, is oxidized and sticks to the particle, which is excreted by the patient in the feces. As a result, the partial pressure of H₂S is decreased which alleviates aforementioned issues.

Therefore, the present invention relates to a (micrometer or thereabouts sized) particle for use to conduct an oxidation or a reduction reaction in a solution in vivo, whereby said particle comprises a metal and/or a metal oxide, is coated with an inert layer -such as a conductive and porous carbon based layer- and releases less metallic ions in said solution compared to a similar but uncoated particle.

More specifically, the present invention relates to a particle as defined above wherein said particle is a microparticle or a nanoparticle.

The present invention further relates to a particle as described above wherein said metal is either iron or manganese and said metal oxide is manganese (IV) oxide, iron (III) oxide or iron (III) hydroxide or any other metal based compounds.

The present invention also relates to a particle as described above wherein said metal oxide is manganese (IV) oxide.

The present invention also relates to a particle as described above wherein said carbon based coating is graphene, graphene oxide, amorphous carbon or diamond-like coating.

The present invention also relates to a carbon coating as described above fabricated by pyrolysis, chemical vapour deposition, hydrothermal carbonization, solvothermal carbonization, microwave synthesis or self-assembly or any other methods known in the art.

More specifically, the present invention discloses a particle as described above wherein said oxidation reaction in a solution in vivo is the oxidation of hydrogen sulfide by manganese (IV) oxide in the gastrointestinal tract of an animal.

The present invention further relates to a particle as describe above whereby said animal is a human and, more specifically, whereby said human is a patient suffering from excessive flatulence or IBS, or whereby said human is a patient suffering from inflammatory bowel disease.

Moreover, the present invention relates to a particle as described above wherein said inflammatory bowel disease is Crohn's disease or ulcerative colitis.

The present invention further relates to a particle as described above wherein said particle is administered as an oral capsule or liquid drinks form, or, as a suppository.

The present invention thus also relates to a method to reduce the amount of hydrogen sulfide in the gastrointestinal tract of a patient comprising administering an effective dose of a particle as described above to a patient in need thereof.

Similarly, the invention also relates to oxidative and reductive conversions of compounds in the gut with the same approach as outlined for the hydrogen sulfide. Particles of zero-valent iron or another metal can be coated with carbon and used to reduce e.g. hydrogen peroxide or other nuisance compounds.

The present invention further relates to a particle as indicated above wherein the surface of the particle is modified by attaching enzymes and/or cofactors and/or redox mediators and/or matrix forming compounds.

Upon modification with enzymes, the particles can be used to perform specific oxidation and or reduction reactions in the gut. An example for this is the immobilization of alcohol dehydrogenase which driven by a cofactor converts ethanol to acetate.

### Detailed description of invention

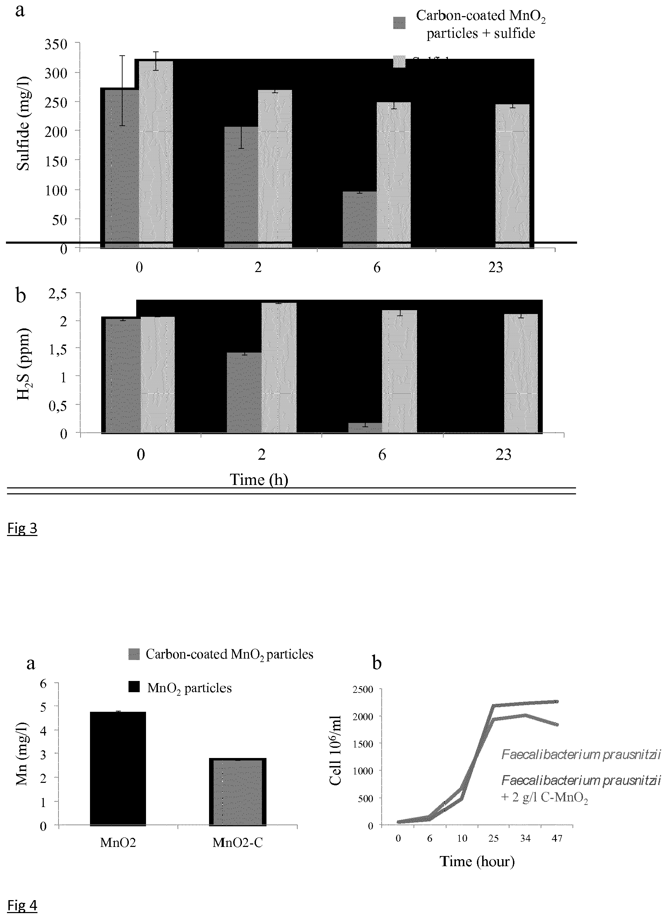

The present invention relates to a particle for use to conduct an oxidation or a reduction reaction in a solution in vivo, whereby said particle comprises a metal and/or a metal oxide, is coated with an inert layer which can be conductive and porous and can consist of carbon or e.g. cellulose acetate and releases less metallic ions in said solution compared to a similar but uncoated particle (i.e. MnO₂; Figure 3b). Inert refers in this context to the absence of a chemical transformation of the coating during the passage through the gut.

The terms' a particle comprising a metal and/or a metal oxide, and, coated with a conductive and porous carbon layer' means a particle wherein said metal is either iron or manganese or another metal and said metal oxide is manganese (IV) oxide, iron (III) oxide or iron (III) hydroxide or another metal (hydr)oxide and wherein said carbon layer coating means graphene, graphene oxide, amorphous carbon or a diamond-like coating. Similarly the carbon could be replaced by other inert, conductive materials such as indium doped tin oxide or cellulose acetate.

The particles can be fabricated by any preferred means known to a skilled person in the art, e.g. precipitation reactions, colloidal synthesis, hydrothermal reaction, solvothermal reaction, chemical vapour deposition, milling, spray based synthesis or self-assembly. The particles and their coating can vary in size, but are typically micrometer or nanometer sized and can thus be denominated as nanoparticles or microparticles.

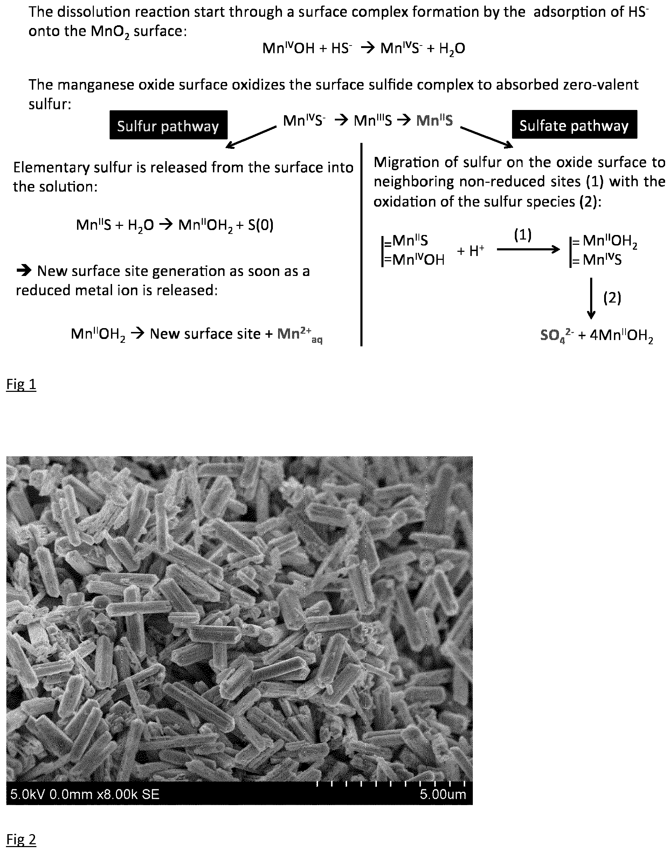

The particles can cause both an oxidative or a reductive reaction in the solution in vivo. Oxidative implies that reduced species in the solution are oxidized by the particle, implying a reduction within the particle. For example, and as outlined in Figure 1, manganese dioxide can be reduced to Mn²⁺, via reaction with sulfide. Similarly, the particles can cause a reduction whereby e.g. zerovalent iron in the particle is converted to Fe²⁺ and Fe³⁺ with e.g. hydrogen peroxide in solution.

During the oxidation and reduction reactions, potentially soluble metal forms are produced. Their release is minimized by the application of the aforementioned coating (Figure 3b) which can be for example verified using atomic adsorption spectroscopy.

The present invention further relates to a particle as described above wherein said metal is either iron or manganese and said metal oxide is manganese (IV) oxide, iron (III) oxide or iron (III) hydroxide. Other metals such as bismuth, cobalt, copper and their derivatives can equally be applied for this purpose.

The present invention also relates to a particle as described above wherein said metal oxide is manganese (IV) oxide (Figure 2).

More specifically, the present invention discloses a particle as described above wherein said oxidation reaction in a solution in vivo is the oxidation of hydrogen sulfide by manganese (IV) oxide in the gastrointestinal tract of an animal. The dissolution reaction start through a surface complex formation by the adsorption of HS- onto MnO2 surface (Figure 1a). The manganese oxide surface oxidizes the surface sulfide complex to absorbed zero-valent sulfur (Figure 1b). Then in sulfur pathway, elementary sulfur is released from the surface into the solution, generated new surface site as soon as a reduced metal ion is released (Figure 1c). In sulfate pathway, sulfur migrate on the oxide surface to neighboring non-reduced sites with the oxidation of the sulfur species (Figure 1d) (Herszage & dos Santos Afonso, 2003).

Typical host animals include humans as well as domestic pets such as dogs and cats, and livestock animals such as cows.

The present invention further relates to a particle as describe above whereby said animal is a human and, more specifically, whereby said human is a patient suffering from flatulence or whereby said human is a patient suffering from inflammatory bowel disease. The terms "a patient suffering from flatulence or whereby said human is a patient suffering from inflammatory bowel disease" are, for example, explained in detail in WO 2006102536.

Moreover, the present invention relates to a particle as described above wherein said inflammatory bowel disease is Crohn's disease or ulcerative colitis.

The present invention further relates to a microparticle as described above wherein said microparticle is administered as an oral solid in the form of a capsule, oral liquid in the form of a drink, or, as an anal suppository.

The present invention thus also relates to a method to reduce the amount of hydrogen sulfide in the gastrointestinal tract of a patient above physiological levels (which an e.g. be above 60 mg/L) comprising administering an effective dose of a particle as described above to a patient in need thereof.

Considering a gut volume of about 1 liter and the need to remove about 30 mg HS⁻ per liter, an effective dose is in the range of 0.15-1.50 g particles per day.

Similarly the present invention also relates to a method to perform reductive reactions in the gut. Compounds such as hydrogen peroxide are harmful to a host. Upon using zerovalent iron or other metals within the core, the particle is able to reduce the levels of such oxidized, harmful compounds.

Finally provided the surface coating is comprised of carbon, enzymes can be covalently bonded to the surface or become immobilized by e.g. the application of a matrix. In combination with cofactors and/or redox mediators, electron supply or draw is possible with the enzymes, enabling them to perform specific catalytic reactions. One example is the conversion of ethanol via partial oxidation using an alcohol dehydrogenase.

The invention will now be further illustrated by the following non-limiting examples.

### Examples

### Example 1: Synthesizing the particles of the present invention

The first reaction step starts from a MnCl2 and KMnO4 suspension which is reacted hydrothermally to form MnO2 nanoparticles. These are then filtered, dried and resuspended by ultrasonication in a glucose suspension followed by a second hydrothermal treatment to form a carbon shell around the particles. These particles are then filtered and dried to form the final product.

### Example 2: removal of hydrogen sulfide by the particles of the present invention in vitro

Figure 3 shows the efficiency of hydrogen sulphide compounds removal present in liquid (a) and gas (b) fraction through a day, in mixed (200 rpm) phosphate buffer (50 mM;pH 5.8). This experiment has been done in 20 ml penicilline bottles in anaerobic condition. At t0, 312.5 mg/l of hydrogen sulfide have been add in two pairs of duplicate bottles and 2 g/l of carbon-coated MnO₂ have been added to one pair of duplicate bottles. Sulfide concentration have been measured using a 930 Compact IC Flex (Metrohm, Switzerland) ion chromatography system and H₂S have been measured using a Compact GC (Global Analyser Solutions, Breda, The Netherlands), equipped with a a Rt-Q-bond pre-column and column. Figure 3 shows that carbonised coated-MnO₂ particles are efficient for sulfide removal after 6 hours experiment and hydrogen sulfide is completely removed within a day.

### Example 3: the particles of the present invention release less metallic ions in solution and have no disturbing effect on commensal bacteria in vitro

Figure 4a shows a similar experiment as example 2 run with non-coated MnO₂ particles and shows that carbon-coated MnO₂ particles allow to reduce by two the total amount of Mn²⁺ released in the environment. Figure 4b represent bacterial cell enumeration of pure bacterial strain (*Faecalibacterium prausnitzii)* growth in dedicated growing medium (M2GSC) with or without addition of carbon-coated MnO₂ particles. Mn²⁺ have been analyzed by means of Atomic Absorption Spectrometry (BRAND, MODEL) and bacterial cell enumeration has been measure using flow cytometry according to De Roy *et al.* (2012). It is shown that well described commensal bacteria, such as *Faecalibacterium prausnitzii,* growth is not disturb by the presence of carbon-coated MnO₂ particles.

### Example 4: Safety assessment of the particles of the present invention in vivo

Wild-type, specific pathogen free, C57BL/6 mice, considered normal control mice, are used to assess initial safety of the coated and uncoated microparticles. Mice are divided into five groups-1) orally gavage uncoated particles, 2) orally gavaged coated particles, 3) uncoated particles incorporated into the food, 4) coated particles incorporated into the food, 5) untreated animals. Male and female adult mice are randomly assigned on one of five groups for a duration of two weeks. Body weight, food/water consumption are measured every other day, and the rodent body score index is used to assess health. Stool samples are collected daily. Upon termination autopsy is performed on all major organs to assess gross inflammation or pathology, while histological analyses of the small intestine, cecum, proximal and distal colon is performed to assess microscopic damage. Blood is collected to assess circulating levels of MnO2.

### References

De Roy, K., Clement, L., Thas, O., Wang, Y., & Boon, N. (2012). Flow cytometry for fast microbial community fingerprinting. water research, 46(3), 907-919.
Herszage, J., & dos Santos Afonso, M. (2003). Mechanism of hydrogen sulfide oxidation by manganese (IV) oxide in aqueous solutions. Langmuir, 19(23), 9684-9692.

## Claims

1. A particle for use to conduct an oxidation or a reduction reaction in a solution in vivo, whereby said particle comprises a metal and/or a metal oxide, is coated with an inert layer and releases less metallic ions in said solution compared to a similar but uncoated particle.

2. A particle according to claim 1 wherein said inert layer is conductive and porous.

3. A particle according to any of claims 1-2 wherein said inert layer is carbon based.

4. A particle according to any of claims 1-3 wherein said particle is a nanoparticle or a microparticle.

5. A particle according to any of claims 1-4 wherein said metal is either iron or manganese and said metal oxide is manganese (IV) oxide, iron (III) oxide or iron (III) hydroxide.

6. A particle according to claim 5 wherein said metal oxide is manganese (IV) oxide.

7. A particle according to any of claims 3-6 where said porous carbon based layer is graphene, few layer graphite, graphene oxide, few layer graphene oxide, reduced graphene oxide, reduced few-layer graphene oxide, amorphous carbon or diamond like coating.

8. A particle according to claim 7 where said porous carbon layers is fabricated by pyrolysis, chemical vapour deposition, hydrothermal carbonization, solvothermal carbonization, microwave synthesis or self-assembly.

9. A particle according to any of claims 1-8 wherein said oxidation reaction in a solution in vivo is the oxidation of hydrogen sulfide by manganese (IV) oxide in the gastrointestinal tract of an animal.

10. A particle according to claim 9 whereby said animal is a human.

11. A particle according to claim 10 whereby said human is a patient suffering from excessive flatulence.

12. A particle according to claim 10 whereby said human is a patient suffering from inflammatory bowel disease.

13. A particle according to claim 12 wherein said inflammatory bowel disease is Crohn's disease or ulcerative colitis.

14. A particle according to any of claims 1-13 wherein said particle is administered as an oral capsule or liquid drinks form or as a suppository.

15. A method to reduce the amount of hydrogen sulfide in the gastrointestinal tract of a patient comprising administering an effective dose of a particle according to any of claims 1-14 to a patient in need thereof.

16. A particle according to any of claims 1-4 wherein said metal is zerovalent iron or cobalt.

17. A particle according to any of claims 1-4 wherein the surface of the particle is modified by attaching enzymes and/or cofactors and/or redox mediators and/or matrix forming compounds.

18. A method using a particle as described in claim 17 where said particle is used to obtain an enzymatic conversion involving an oxidation or a reduction reaction.
